# EUROPEAN PATENT APPLICATION

(11) **EP 4 674 941 A1**
(43) Date of publication of application: **07.01.2026**
(21) Application number: 23925325.5
(22) Date of filing: 02.03.2023
(51) Int. Cl.: C12M 1/00, C12N 15/10

(54) **NUCLEIC ACID EXTRACTION DEVICE AND NUCLEIC ACID EXTRACTION METHOD**

(71) Applicant: HITACHI HIGH-TECH CORPORATION, Tokyo 105-6409 (JP)
(72) Inventor: FUJIOKA Michiru, Tokyo 105-6409 (JP); MITSUYAMA Toshifumi, Tokyo 100-8280 (JP); FUJIOKA Kaoru, Tokyo 105-6409 (JP)
(74) Representative: Strehl & Partner mbB
(86) International application number: PCT/JP2023/007862
(87) International publication number: WO 2024/180770

(57) **Abstract**

The purpose of the present invention is to provide a technology that can efficiently conduct elution and stirring even when the liquid amount of a specimen solution containing nucleic acid and magnetic particles is abundant and the liquid amount eluting therefrom is less. A nucleic acid extraction device according to the present invention comprises a large-diameter first magnetic rod protection cover and a small-diameter second magnetic rod protection cover, and involves: stirring a sample by using the first magnetic rod protection cover; and replacing the first magnetic rod protection cover with the second magnetic rod protection cover and inserting a magnetic rod in the second magnetic rod protection cover to recover magnetic particles in the sample (see fig. 4).

## Description

### Technical Field

The present invention relates to a technique for extracting a nucleic acid from a sample containing the nucleic acid and magnetic particles.

### Background Art

In recent years, information obtained by nucleic acid analysis such as cancer genomic testing using a next-generation sequencing (NGS) system has been used in various fields such as medical care, clinical testing, pharmaceutical industries, and food industries. In such nucleic acid analysis, nucleic acid extraction from various biological samples such as blood, tissue, and a cultured cell is essential preprocessing.

As nucleic acid extraction methods, a method based on a property that a nucleic acid binds to silica in the presence of a chaotropic agent and a method based on a property that a nucleic acid binds to silica in the presence of an organic solvent are generally used instead of methods using phenol, chloroform, and the like, which are hazardous organic solvents. Using these methods, a nucleic acid extraction method using a nucleic acid capturing tip including a solid phase containing silica as a nucleic acid capturing carrier, and a method using magnetic beads (nucleic acid capturing carrier) whose surface is covered with silica have been reported. These methods include a step of binding the nucleic acid to the nucleic acid capturing carrier and an elution step of eluting the nucleic acid from the nucleic acid capturing carrier using an elution liquid.

In the method using the magnetic beads, after the elution step, the magnetic beads are recovered from the elution liquid using a magnet. As an example, there is a method of aspirating the elution liquid containing the magnetic beads into a dispensing tip, retaining the magnetic beads in the dispensing tip using the magnet, and discharging only the elution liquid from the dispensing tip. As another example, there is a method of recovering the magnetic beads from the elution liquid by inserting a rod-shaped magnet (which may be covered with a cover) into the elution liquid containing the magnetic beads.

PTL 1 discloses, as a method for efficiently collecting magnetic beads, a method of collecting magnetic beads on a wall of a container first and then recovering the magnetic beads using a magnetic rod. PTL 2 discloses an example in which a nucleic acid is extracted from a specimen having a large liquid amount (2 mL) through a procedure shown in FIG. 23 using a thick magnetic comb and a thin magnetic comb. PTL 3 discloses a technique of implementing collection of a magnetic body from a magnetic body containing liquid not on the side of a container containing a specimen but using a dispenser that aspirates and discharges the magnetic body containing liquid.

### Citation List

### Patent Literature

PTL 1: US6020211
PTL 2: US2022/0176369A1
PTL 3: JP3115501B

### Summary of Invention

### Technical Problem

In recent years, while a liquid amount of specimen (sample liquid) to be measured increases, a liquid amount of eluate obtained by eluting a nucleic acid from the specimen may decrease as compared to the related art. This is to concentrate the eluate and thus bring the entire eluate into subsequent steps. When an eluting liquid amount is small with respect to an amount of input specimen, it is required to use a dispensing tip having a small inner diameter in order to recover the small amount of eluate in the method of aspirating and discharging the magnetic body containing liquid using the dispenser. However, due to this, an increased amount of magnetic beads for efficiently recovering the nucleic acid from a large amount of specimen solution is prone to clogging. Meanwhile, in the method using the magnetic rod, when a thick magnetic rod is used, stirring efficiency decreases in a step of stirring the small amount of eluate, and when a thin magnetic rod is used, it is difficult to stir the large amount of specimen solution.

PTL 1 discloses only a case where a thin and small magnetic rod is used when a large amount of specimen is used, and thus it is conceivable that the above-described problem related to the magnetic rod exists. In other words, it is considered that the same literature does not assume stirring a large amount of liquid added to a container larger than an outer diameter of the magnetic rod or collecting magnetic beads from the large amount of liquid.

As shown in FIG. 23 in PTL 2, when elution is first performed using a thick magnetic rod protection cover and then elution is performed again using a thin magnetic rod protection cover, adsorption (magnetic collection) and elution of DNA and magnetic particles are each implemented twice, and thus a DNA recovery rate decreases. This is because a proportion of the DNA adsorbed to the magnetic particles is not 100% and the elution is also not 100%. In addition, there is a problem that the recovery rate decreases since the adsorption and elution are repeated. For example, when the recovery rate is 80%, the recovery rate after two repetitions is 80% × 80% = 64%. In addition, since the DNA and the magnetic particles are each adsorbed and eluted twice, a long time is required.

In PTL 3, since the magnetic body containing liquid is aspirated and discharged using the dispenser, it is conceivable that the above-described problem related to the dispensing tip exists.

The invention was made in view of the problems described above, and an object thereof is to provide a technique that can efficiently implement elution and stirring even when a liquid amount of specimen solution containing a nucleic acid and magnetic particles is large and an amount of liquid eluting therefrom is small.

### Solution to Problem

A nucleic acid extraction device according to the invention includes a thick first magnetic rod protection cover and a thin second magnetic rod protection cover, a sample is stirred using the first magnetic rod protection cover, the first magnetic rod protection cover is replaced with the second magnetic rod protection cover and then a magnetic rod is inserted into the second magnetic rod protection cover to recover magnetic particles in the sample.

### Advantageous Effects of Invention

According to the nucleic acid extraction device according to the invention, elution and stirring can be efficiently implemented even when a liquid amount of specimen solution containing a nucleic acid and magnetic particles is large and an amount of liquid eluting therefrom is small. Other problems, configurations, advantages, and the like of the invention will become apparent from the following description of embodiments.

### Brief Description of Drawings

[FIG. 1A] FIG. 1A is a view illustrating a problem when a thick magnetic rod protection cover is used.
[FIG. 1B] FIG. 1B is a view illustrating a problem when a thin magnetic rod protection cover is used.
[FIG. 2] FIG. 2 is a perspective view of a nucleic acid extraction device 1 according to a first embodiment.
[FIG. 3] FIG. 3 is a side view revealing the inside of the nucleic acid extraction device 1.
[FIG. 4] FIG. 4 is a view illustrating a step of extracting a nucleic acid from a sample by the nucleic acid extraction device 1.
[FIG. 5] FIG. 5 is a view illustrating a step of extracting the nucleic acid from the sample by the nucleic acid extraction device 1 according to a second embodiment.
[FIG. 6] FIG. 6 is a view illustrating a step of extracting the nucleic acid from the sample by the nucleic acid extraction device 1 according to a third embodiment.

### Description of Embodiments

### <Problems in Related Art >

Hereinafter, problems in the related art will be described first, and then embodiments of the invention will be described. Since the problem in PTL 2 has been described above, a specific example of PTLs 1 and 3 will be described here.

The method of aspirating and discharging the magnetic body containing liquid using the dispenser as in PTL 3 has the following problems.
- When an amount of eluting liquid is small with respect to an amount of input specimen, a proportion of an eluate adhering to magnetic beads increases, and it is difficult to recover the eluate. This is because an amount of eluate is relatively small, and thus the relative proportion of the adhering eluate increases.
- In a case where the amount of eluate is small, when the liquid is aspirated and discharged using the dispenser, there is a possibility that an inner diameter of a dispensing tip is excessively large, only air passes, and stirring cannot be performed.
- For the above reasons, it is required to use a dispensing tip having a small inner diameter to recover the small amount of eluate. However, in this case, the magnetic beads are prone to clogging. In particular, when the amount of input specimen increases, it is required to increase the magnetic beads in order to efficiently capture a nucleic acid, and thus clogging is likely to occur.

The method using the magnetic rod as in PTL 2 has the following problems.
- When the amount of input specimen increases, a thick and large magnetic rod protection cover (a cover for protecting the magnetic rod) is used to facilitate stirring. Since the magnetic rod protection cover is excessively large with respect to a small amount of eluate at the time of elution, stirring efficiency decreases. Further, since the magnetic rod protection cover is relatively large with respect to a volume of the small amount of eluate, a proportion of the eluate adhering to the magnetic rod protection cover increases, and recovery efficiency of the eluate decreases.
- On the other hand, when a thin and small magnetic rod protection cover is used, a vortex flow cannot be generated in a large amount of specimen solution, and stirring becomes difficult (a new mechanism is required for stirring). In addition, magnetic collection efficiency decreases.

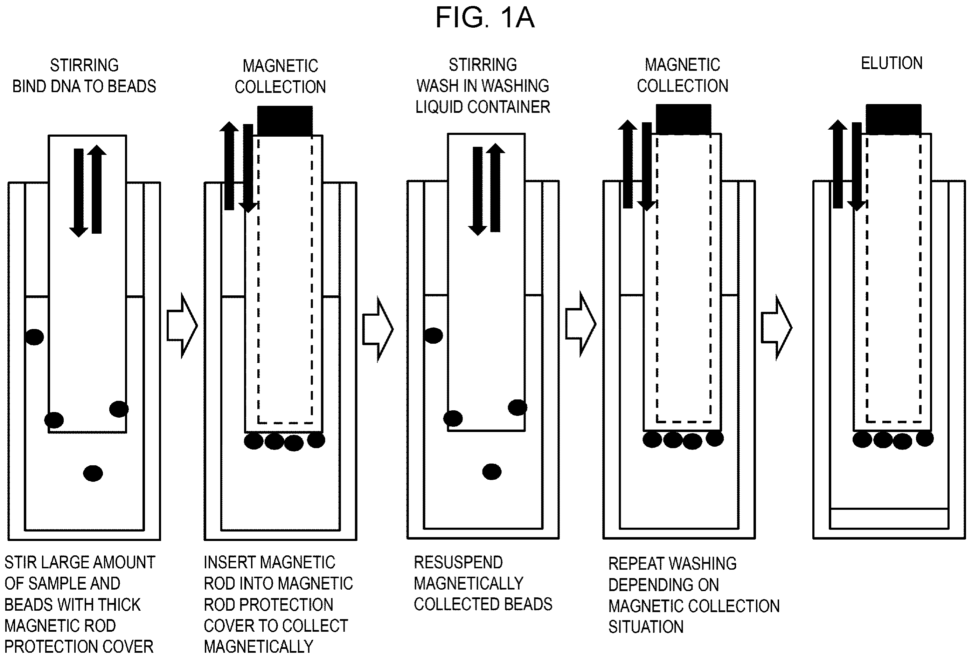

FIG. 1A is a view illustrating the problem when the thick magnetic rod protection cover is used. Since the magnetic rod protection cover is relatively large with respect to the volume of the eluate, stirring efficiency is low. When an up-and-down motion of the magnetic rod protection cover is strongly performed in order to implement thorough stirring, an amount of eluate adhering to a side surface of the magnetic rod protection cover increases, and recovery efficiency of the eluate decreases. Therefore, in the method using the magnetic rod, it is not desirable to use a thick magnetic rod protection cover as shown in FIG. 1A.

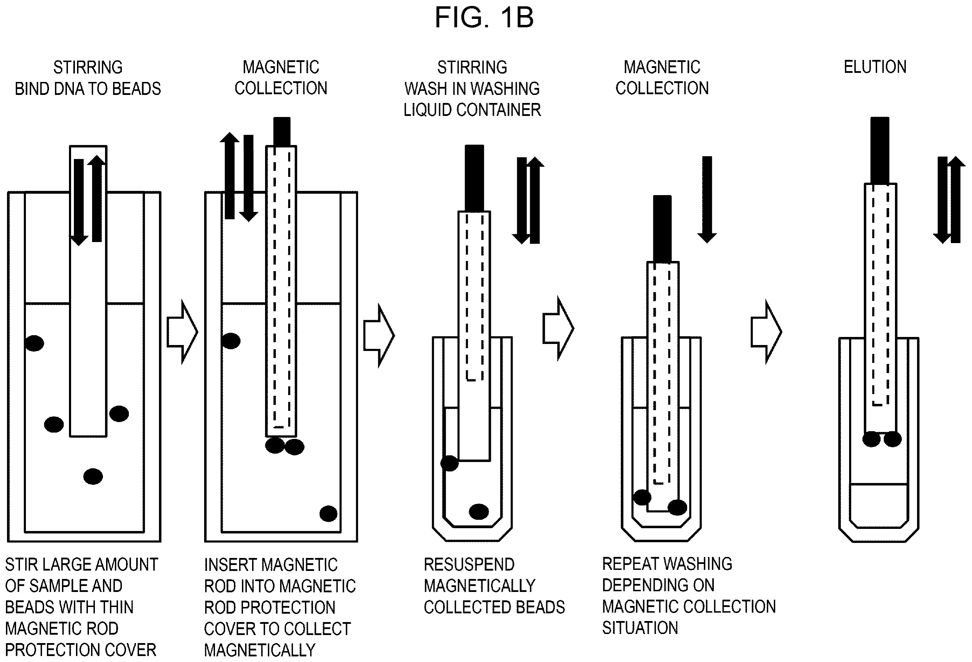

FIG. 1B is a view illustrating the problem when the thin magnetic rod protection cover is used. When the thin magnetic rod protection cover is used, it is difficult to stir the large amount of specimen solution, and therefore, it is required to use a mechanism other than the magnetic rod protection cover for stirring. In addition, since the magnetic rod is also as thin as the magnetic rod protection cover, efficiency of recovering magnetic beads decreases.

### <First Embodiment>

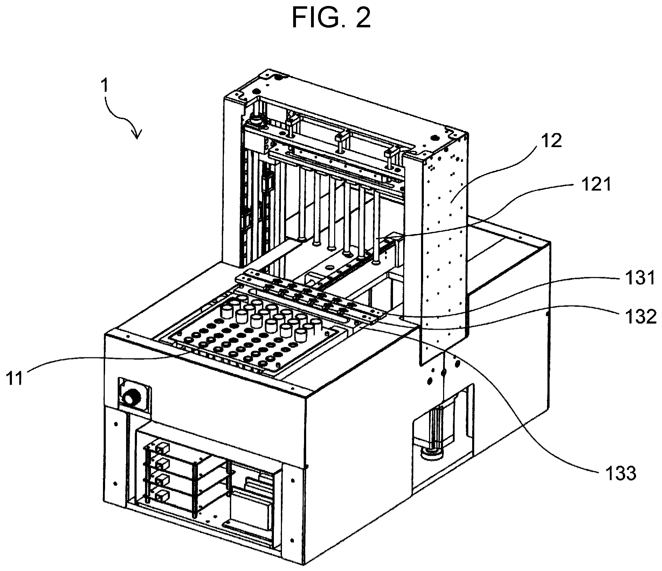

FIG. 2 is a perspective view of a nucleic acid extraction device 1 according to a first embodiment of the invention. The nucleic acid extraction device 1 is a device for extracting a nucleic acid from a sample containing the nucleic acid and magnetic particles. The nucleic acid extraction device 1 includes an X-axis module 11, a Z-axis module 12 (a mechanism for moving a magnetic rod protection cover and a magnetic rod), magnetic rod protection covers 131 and 132, and a bottom magnet 14 to be described later.

The X-axis module 11 includes a container 111 that contains the sample and a container holding mechanism 112 that holds the container 111. The X-axis module 11 can move the container 111 and the container holding mechanism 112 in a horizontal direction (X-axis direction).

The Z-axis module 12 includes a magnetic rod 121 and can move the magnetic rod 121 up and down in a vertical direction (Z-axis direction). The Z-axis module 12 further includes a mechanism that can attach and detach the magnetic rod protection covers 131 and 132 to and from the magnetic rod 121. Accordingly, the magnetic rod protection covers 131 and 132 can be replaced with each other as will be described later. The X-axis module 11 moves the magnetic rod protection cover 131 or 132 below the magnetic rod 121, and the Z-axis module 12 moves the magnetic rod 121 up and down, whereby the magnetic rod protection cover 131 or 132 can be attached to and detached from the magnetic rod 121. Further, the X-axis module 11 moves the container 111 below the magnetic rod 121 (to which the magnetic rod protection cover is attached) and moves the magnetic rod 121 up and down, thereby enabling a liquid in the container 111 to be stirred by the magnetic rod protection cover.

The magnetic rod protection cover 131 is a member similar to the thin magnetic rod protection cover described in FIG. 1B. The magnetic rod protection cover 132 is a member similar to the thick magnetic rod protection cover described in FIG. 1A. That is, a diameter of the magnetic rod protection cover 131 is smaller than a diameter of the magnetic rod protection cover 132. It is not necessarily required that the entire magnetic rod protection cover 131 be thin. It is sufficient that at least a diameter of a part of the magnetic rod protection cover 131 immersed in the sample is smaller than a diameter of a part of the magnetic rod protection cover 132 immersed in the sample. Two types of magnetic rod protection covers are shown here, and for example, a third type of magnetic rod protection cover may be placed at a position of reference numeral 133 and may be selected according to a specimen liquid amount or the container. FIG. 2 shows a state in which the protection cover of the third type is attached to the magnetic rod 121.

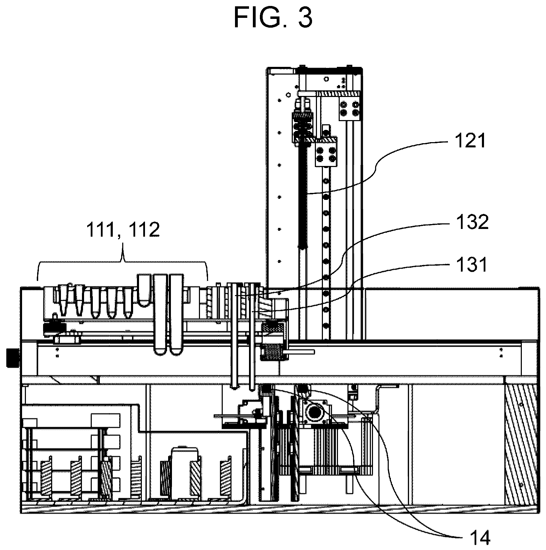

FIG. 3 is a side view revealing the inside of the nucleic acid extraction device 1. The container holding mechanism 112 holds a plurality of types of containers 111. For example, a plurality of types of containers 111 such as a container 111 that holds a sample liquid and a container 111 that holds a reagent are held.

The magnetic rod protection cover 132 is desirably closer to the container 111 than to the magnetic rod protection cover 131. On the other hand, when the magnetic rod protection cover 131 is placed at a position closer to the container 111, the magnetic rod protection cover 132 passes over the magnetic rod protection cover 131 in a step to be described later, and a droplet may fall from the magnetic rod protection cover 132 and contaminate the magnetic rod protection cover 131.

The nucleic acid extraction device 1 further includes the bottom magnet 14. The bottom magnet 14 is located below the container 111 when the container 111 moves below the magnetic rod 121. The bottom magnet 14 can be moved up and down by, for example, the Z-axis module 12. When the bottom magnet 14 approaches a bottom surface of the container 111, magnetic beads can be magnetically collected at the bottom surface of the container 111. A plurality of types of bottom magnets 14 may be provided and selected, such as one used for a large container 111 and one used for a small container 111, or only one bottom magnet 14 may be used.

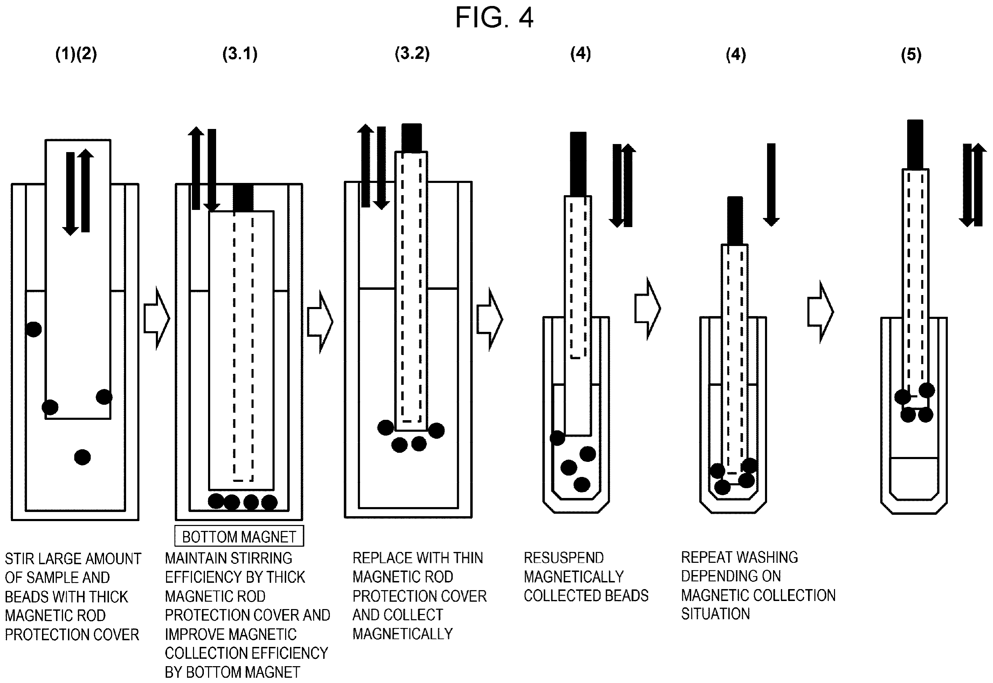

FIG. 4 is a view illustrating a step of extracting the nucleic acid from the sample by the nucleic acid extraction device 1. Each step in FIG. 4 will be described below.
(1) Addition of beads: magnetic beads (magnetic particles) are added to a large amount of sample solution (specimen: whole blood, plasma, serum, and the like). Further, a reagent for promoting binding between the magnetic beads and a biological substance (DNA, antibody, or the like) to be captured is added as necessary.
(2) Stirring: the sample solution is stirred using the magnetic rod protection cover 132 (thick magnetic rod protection cover) such that the magnetic beads and the biological substance are bound. For example, the Z-axis module 12 moves the magnetic rod protection cover 132 up and down in the sample solution. When the magnetic beads and the biological substance are sufficiently bound, the process proceeds to the next step.
(3.1) Magnetic collection: the magnetic rod 121 is inserted into the magnetic rod protection cover 132. A diameter of the magnetic rod 121 is substantially the same as that of the magnetic rod protection cover 131 (thin magnetic rod protection cover). The magnetic rod protection cover 132 into which the magnetic rod 121 is inserted is moved, for example, up and down to stir the sample solution and capture the magnetic beads. Using the thick magnetic rod protection cover, a high stirring effect can be obtained. More preferably, by moving the magnetic rod 121 up and down while bringing the bottom magnet 14 close to a wall (bottom) of the container, the magnetic beads can be captured at two positions of the container wall (bottom) and the magnetic rod 121, and capturing efficiency can be improved. Thereafter, after the magnetic rod 121 is brought close to the container wall (bottom), only the magnetic rod 121 is taken out from the magnetic rod protection cover 132, and then the magnetic rod protection cover 132 is pulled up from the sample solution. As described above, the magnetic beads are magnetically collected at the container bottom.
(3.2) Magnetic collection: after the magnetic rod 121 is inserted into the magnetic rod protection cover 131 (thin magnetic rod protection cover), the magnetic rod protection cover 131 is brought close to the container bottom. When the bottom magnet 14 is separated from the container bottom in this state, the magnetic beads are magnetically collected around the magnetic rod protection cover 131.
(4) Washing: the magnetic beads to which the nucleic acid is bound are washed, and a nonspecifically bound substance is removed from a nucleic acid capturing carrier. In the magnetic collection step (3.1 and 3.2), since the magnetic beads are magnetically collected to the magnetic rod protection cover 131, it is possible to wash the magnetic beads in a container smaller than the sample container containing a large amount of specimen or in a container having a shape corresponding to a shape of the magnetic rod protection cover 131. By using the small container or the container shape corresponding to the shape of the magnetic rod protection cover 131, washing can be performed with a small amount of washing liquid, and thus cost is reduced. The container shown in step (4) in FIG. 4 is a washing liquid container thereof and is held on the container holding mechanism 112. After the magnetic rod protection cover 131 and the magnetic beads are washed, an alcohol component and the like are dried such that the washing liquid is not mixed into a nucleic acid elution liquid in a next step. A drying method is not limited, and the alcohol component evaporates by causing the magnetic rod protection cover 131 into which the magnetic rod 121 is inserted to stand by or move up and down above the washing container.
(4) Washing: supplement 1: a container suitable for washing, a washing liquid amount, and the number of times of washing may be determined depending on a purpose. The same applies not only to the washing step but also to a subsequent elution step. For example, in the washing step, since the magnetic beads and the magnetic rod protection cover 131 are washed, a bottom shape of the container is set to a round bottom structure or the like such that the solution easily flows. In the elution step, since the nucleic acid is eluted with a small amount of eluate, a small container inner diameter close to an outer diameter of the magnetic rod protection cover 131 is adopted similarly to the washing liquid container.
(4) Washing: supplement 2: a washing reagent is not particularly limited, and may be, for example, a reagent that can remove the reagent added in step 1 and impurities from the nucleic acid capturing carrier while maintaining binding of the nucleic acid to the nucleic acid capturing carrier. For example, an organic compound such as a lower alcohol or a lower-molecular-weight ketone can be used. As the washing reagent, ethanol, isopropanol, or the like can be used, and ethanol having a concentration of 70% or more is particularly preferably used.
(5) The nucleic acid bound to the magnetic beads is eluted from the magnetic beads. Specifically, the magnetic rod protection cover 131 capturing the magnetic beads to which the nucleic acid is bound is immersed in a container containing the nucleic acid elution liquid, and is stirred by up-and-down movement. Accordingly, the nucleic acid is recovered in the nucleic acid elution liquid. The magnetic rod 121 may be removed from the magnetic rod protection cover 131 during stirring. In this case, after stirring, the magnetic rod 121 is inserted again into the magnetic rod protection cover 131 to magnetically collect the magnetic beads, and the magnetic rod protection cover 131 is pulled up. The container containing the nucleic acid elution liquid may be smaller than the washing container. Accordingly, efficiency of stirring the nucleic acid elution liquid by the magnetic rod protection cover 131 is improved. A container shown in step (5) in FIG. 4 is the nucleic acid elution liquid container and is held on the container holding mechanism 112.

### <First Embodiment: Summary>

In the nucleic acid extraction device 1 according to the first embodiment, the thick magnetic rod protection cover (magnetic rod protection cover 132) is used to facilitate stirring even when the amount of input specimen increases, and on the other hand, the "thin magnetic rod protection cover + thin magnetic rod" are used for the small liquid amount at the time of elution. Accordingly, it is possible to recover the nucleic acid with a high concentration without reducing the stirring efficiency. Further, a small amount of eluate droplets adhering to the magnetic rod protection cover is limited, and eluate recovery efficiency is not reduced. In other words, by using the nucleic acid extraction device 1, all (or most) of the nucleic acid can be eluted from the magnetic beads by reliably stirring the magnetic beads and the nucleic acid elution liquid, and the nucleic acid can be dissolved with a high concentration.

### <Second Embodiment>

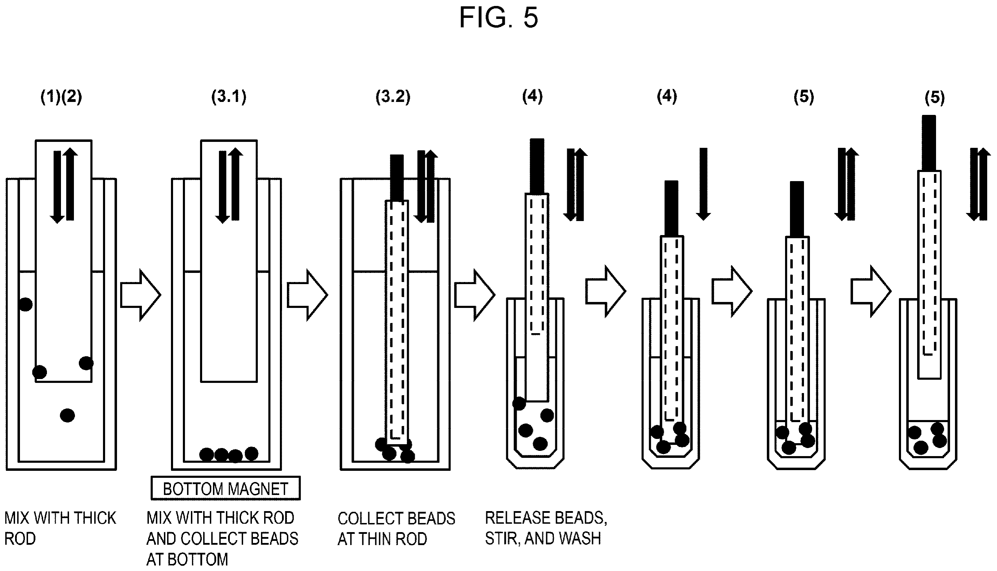

FIG. 5 is a view illustrating a step of extracting the nucleic acid from the sample by the nucleic acid extraction device 1 according to a second embodiment of the invention. A difference from the first embodiment is the magnetic collection step 3.1. In the step 3.1, the magnetic beads may be magnetically collected only by placing the bottom magnet 14 at the bottom of the sample container without inserting the magnetic rod 121 into the magnetic rod protection cover 132. Others are the same as those in the first embodiment. In the step shown in FIG. 5, there is a possibility that the efficiency of magnetically collecting the magnetic beads in the step 3.1 is lower than that in the first embodiment. On the other hand, since a frequency of driving the magnetic rod 121 is reduced, wear of the magnetic rod 121 (and the Z-axis module 12) can be reduced. The step in the second embodiment is useful if wear reduction is prioritized. Further, since the magnetic collection efficiency is improved by the presence of the bottom magnet 14, a structure of the magnetic rod 121 can be a structure specialized for subsequent washing and elution steps in which the liquid amount is small (for example, a thinner structure).

### <Third Embodiment>

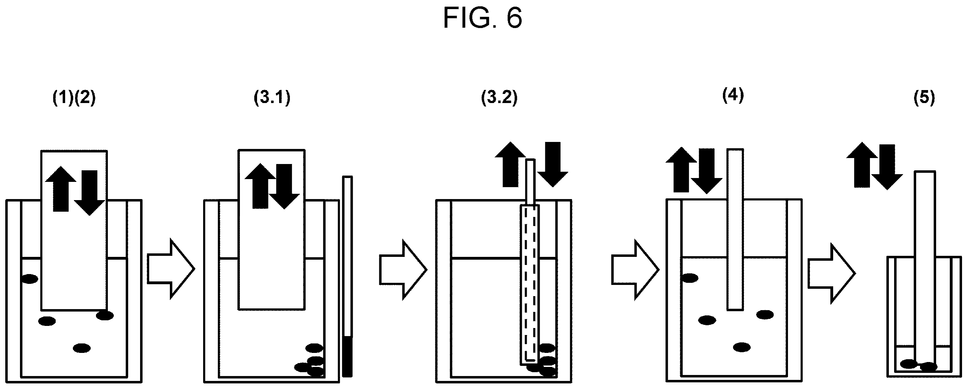

FIG. 6 is a view illustrating a step of extracting the nucleic acid from the sample by the nucleic acid extraction device 1 according to a third embodiment of the invention. A difference from the first embodiment is the magnetic collection step 3.1. In the step 3.1, the magnetic rod 121 is not inserted into the magnetic rod protection cover 132, and the bottom magnet 14 is not used. Instead, the magnetic rod 121 is brought close to a side surface of the sample container. During that time, the solution is stirred by the thick magnetic rod protection cover to magnetically collect the magnetic beads inside the container to which the magnetic rod 121 is brought close.

The step 3.2 and subsequent steps are the same as those in the first embodiment, and since the magnetic beads are accumulated at the side surface of the sample container, it is required to bring the magnetic rod protection cover 131 (and the magnetic rod 121 inserted therein) close to the vicinity of the side surface of the container. Others are the same as those in the first embodiment.

The step shown in FIG. 6 may require a longer time for magnetic collection than in the first embodiment, but has an advantage that it is not required to newly prepare a magnet other than the magnetic rod (that is, the bottom magnet 14) at the bottom of the sample container. Accordingly, the bottom magnet 14 and a mechanism for controlling movement thereof can be omitted.

### <Fourth Embodiment>

In the above embodiments, the switching between the magnetic rod protection covers 132 and 131 is not necessarily performed in the magnetic collection steps 3.1 and 3.2, and may be performed in the washing step. In this case, the magnetic beads are magnetically collected by the magnetic rod protection cover 132 (thick magnetic rod protection cover), and the magnetic rod protection cover 132 holding the magnetic beads is washed. After washing, the magnetic beads adhering to the magnetic rod protection cover 132 are magnetically collected to the container bottom surface by the bottom magnet 14. The magnetic beads at the container bottom surface are magnetically collected again by the magnetic rod protection cover 131 and the magnetic rod 121. The subsequent steps are the same as those in the first embodiment. The above procedure is different from the first embodiment in that the magnetic rod protection cover 132 is replaced with the magnetic rod protection cover 131 after washing. In this procedure, the washing container and the elution container are larger than those in the first embodiment, and a degree of freedom in timing for replacing the magnetic rod protection cover is improved as compared to the first embodiment.

Similarly, the procedure of replacing the magnetic rod protection cover after washing can also be used in the procedure of the second embodiment. In the magnetic collection step 3.1, the magnetic beads magnetically collected first at the container bottom surface are magnetically collected again by the magnetic rod protection cover 132 (thick magnetic rod protection cover), and the magnetic rod protection cover 132 holding the magnetic beads is washed. After washing, the magnetic beads adhering to the magnetic rod protection cover 132 are magnetically collected to the container bottom surface by the bottom magnet 14. The magnetic beads at the container bottom surface are magnetically collected again by the magnetic rod protection cover 131 and the magnetic rod 121. The subsequent steps are the same as those in the first embodiment.

### <Modifications of Invention>

In the above embodiments, the nucleic acid extracted from the sample can be subjected to a subsequent analysis step. Analysis using the nucleic acid solution is not particularly limited, and examples thereof include sequencing based on a next-generation sequencer, DNA microarray, electrophoresis, and high performance liquid chromatography-mass spectrometry.

In the above embodiments, an example in which the nucleic acid is adhered to the magnetic beads as the target substance and extracted has been described, but even when another target substance is adhered to the magnetic beads and recovered, the same device structure and the same extraction step as those of the nucleic acid extraction device 1 can be used by appropriately changing a composition of the sample liquid or the elution liquid according to the target substance.

### Reference Signs List

1: nucleic acid extraction device
121: magnetic rod
131: magnetic rod protection cover
132: magnetic rod protection cover
14: bottom magnet

## Claims

1. A nucleic acid extraction device for extracting a nucleic acid from a sample containing the nucleic acid and a magnetic particle, the nucleic acid extraction device comprising:
a first magnetic rod protection cover configured to stir the sample;
a second magnetic rod protection cover having a portion thinner than a part of the first magnetic rod protection cover immersed in the sample;
a magnetic rod insertable into the first magnetic rod protection cover and the second magnetic rod protection cover; and
a mechanism configured to move the first magnetic rod protection cover, the second magnetic rod protection cover, and the magnetic rod, wherein
the mechanism stirs the sample using the first magnetic rod protection cover,
after the stirring, the mechanism replaces the first magnetic rod protection cover with the second magnetic rod protection cover and then immerses the portion in the sample,
the mechanism inserts the magnetic rod into the second magnetic rod protection cover immersed in the sample, and
the mechanism pulls up, from the sample, the second magnetic rod protection cover into which the magnetic rod is inserted, and thus extracts, from the sample, the magnetic particle adhering to the second magnetic rod protection cover due to a magnetic force of the magnetic rod and the nucleic acid adhering to the magnetic particle.

2. The nucleic acid extraction device according to claim 1, further comprising:
a magnet placeable at a bottom surface of a container that contains the sample, wherein
the mechanism moves the magnet to the bottom surface of the container after the stirring,
when the magnet is placed at the bottom surface of the container, the mechanism inserts the magnetic rod into the first magnetic rod protection cover, brings the magnetic rod close to a bottom surface of the first magnetic rod protection cover, and thus magnetically collects the magnetic particle at the bottom surface of the container,
the mechanism takes out the magnetic rod from the first magnetic rod protection cover, further takes out the first magnetic rod protection cover from the sample, and then immerses the second magnetic rod protection cover in the sample to replace the first magnetic rod protection cover with the second magnetic rod protection cover, and
the mechanism pulls up the second magnetic rod protection cover from the sample after removing the magnet from the bottom surface of the container.

3. The nucleic acid extraction device according to claim 1, further comprising:
a magnet placed at a bottom surface of a container that contains the sample, wherein
the mechanism moves the magnet to the bottom surface of the container without inserting the magnetic rod into the first magnetic rod protection cover, and thus magnetically collects the magnetic particle at the bottom surface of the container,
the mechanism replaces the first magnetic rod protection cover with the second magnetic rod protection cover without inserting the magnetic rod into the first magnetic rod protection cover, and
the mechanism pulls up the second magnetic rod protection cover from the sample after removing the magnet from the bottom surface of the container.

4. The nucleic acid extraction device according to claim 1, wherein
the mechanism brings the magnetic rod close to a side surface of a container that contains the sample to magnetically collect the magnetic particle at the side surface of the container after the stirring or during the stirring, and
the mechanism replaces the first magnetic rod protection cover with the second magnetic rod protection cover after bringing the magnetic rod close to the container.

5. The nucleic acid extraction device according to claim 1, further comprising:
a magnet placed at a bottom surface of a container that contains the sample, wherein
the mechanism moves the magnet to the bottom surface of the container after the stirring,
when the magnet is placed at the bottom surface of the container, the mechanism inserts the magnetic rod into the first magnetic rod protection cover, brings the magnetic rod close to a bottom surface of the first magnetic rod protection cover, and thus magnetically collects the magnetic particle at the bottom surface of the container,
the mechanism supplies a washing liquid to the first magnetic rod protective cover to which the magnetic particle adheres, to wash the magnetic particle,
when the magnet is placed at the bottom surface of the container after the washing, the mechanism brings the first magnetic rod protection cover, into which the magnetic rod is inserted, close to the bottom surface of the container, and
the mechanism takes out the magnetic rod from the first magnetic rod protection cover, further takes out the first magnetic rod protection cover from the sample, and then immerses the second magnetic rod protection cover in the sample to replace the first magnetic rod protection cover with the second magnetic rod protection cover.

6. The nucleic acid extraction device according to claim 1, further comprising:
a magnet placed at a bottom surface of a container that contains the sample, wherein
after the stirring, the mechanism moves the magnet to the bottom surface of the container without inserting the magnetic rod into the first magnetic rod protection cover, and thus magnetically collects the magnetic particle at the bottom surface of the container,
the mechanism supplies a washing liquid to the first magnetic rod protective cover to which the magnetic particle adheres, to wash the magnetic particle,
when the magnet is placed at the bottom surface of the container after the washing, the mechanism brings the first magnetic rod protection cover, into which the magnetic rod is inserted, close to the bottom surface of the container, and
the mechanism takes out the magnetic rod from the first magnetic rod protection cover, further takes out the first magnetic rod protection cover from the sample, and then immerses the second magnetic rod protection cover in the sample to replace the first magnetic rod protection cover with the second magnetic rod protection cover.

7. The nucleic acid extraction device according to claim 1, wherein
a maximum diameter of the portion of the second magnetic rod protection cover is smaller than a diameter of the part of the first magnetic rod protection cover immersed in the sample.

8. The nucleic acid extraction device according to claim 1, further comprising:
a holding stage where a container containing the sample is placed, wherein
when the first magnetic rod protection cover and the second magnetic rod protection cover are not immersed in the sample, the first magnetic rod protection cover is placed at a position closer to the holding stage than the second magnetic rod protection cover.

9. The nucleic acid extraction device according to claim 1, wherein
the mechanism supplies a washing liquid to the second magnetic rod protection cover to which the magnetic particle adheres, to wash the magnetic particle, and
after the washing, the mechanism supplies a nucleic acid elution liquid to the second magnetic rod protection cover to recover the nucleic acid into the nucleic acid elution liquid.

10. The nucleic acid extraction device according to claim 9, further comprising:
a washing liquid container configured to contain the washing liquid, wherein
a volume of the washing liquid that can be contained in the washing liquid container is smaller than a volume of the sample that can be contained in a sample container that contains the sample.

11. The nucleic acid extraction device according to claim 9, further comprising:
a nucleic acid elution liquid container configured to contain the nucleic acid elution liquid, wherein
a volume of the nucleic acid elution liquid that can be contained in the nucleic acid elution liquid container is smaller than a volume of the sample that can be contained in a sample container that contains the sample.

12. A nucleic acid extraction method for extracting a nucleic acid from a sample containing the nucleic acid and a magnetic particle, the nucleic acid extraction method comprising:
a step of stirring the sample using a first magnetic rod protection cover for stirring the sample;
a step of replacing, after the stirring, the first magnetic rod protection cover with a second magnetic rod protection cover having a portion thinner than a part of the first magnetic rod protection cover immersed in the sample, and immersing the second magnetic rod protection cover in the sample;
a step of inserting a magnetic rod into the second magnetic rod protection cover immersed in the sample; and
a step of pulling up the second magnetic rod protection cover from the sample and thus extracting, from the sample, the magnetic particle adhering to the second magnetic rod protection cover due to a magnetic force of the magnetic rod and the nucleic acid adhering to the magnetic particle.
